# EUROPEAN PATENT SPECIFICATION

(11) **EP 3 391 049 B1**
(45) Date of publication and mention of the grant of the patent: **26.02.2020**
(21) Application number: 16826300.2
(22) Date of filing: 16.12.2016
(51) Int. Cl.: G01N 33/533, G01N 33/542

(54) **A METHOD OF DETERMINING THE ABUNDANCE OF A TARGET MOLECULE IN A SAMPLE**
VERFAHREN ZUR BESTIMMUNG DER MENGE EINES ZIELMOLEKÜLS IN EINER PROBE
PROCÉDÉ PERMETTANT DE DÉTERMINER L'ABONDANCE D'UNE MOLÉCULE CIBLE DANS UN ÉCHANTILLON

(30) Priority: 18.12.2015 EP 15201419
(43) Date of publication of application: 24.10.2018
(73) Proprietor: Valitacell Limited, Blackrock, Co. Dublin (IE)
(72) Inventor: CLIFFORD, Jerry, Tralee, Co. Kerry (IE); THOMPSON, Ben, Sheffield South Yorkshire S3 7BG (GB); BYRNE, Hannah, Castletown Celbridge, Co. Kildare (IE)
(74) Representative: Purdylucey Intellectual Property
(86) International application number: PCT/EP2016/081585
(87) International publication number: WO 2017/103210

(56) References cited:
- MIN CHEN ET AL: "A novel multiplexed fluorescence polarisation immunoassay based on a recombinant bi-specific single-chain diabody for simultaneous detection of fluoroquinolones and sulfonamides in milk", FOOD ADDITIVES & CONTAMINANTS: PART A, vol. 31, no. 12, 13 November 2014 (2014-11-13), pages 1959-1967, XP055262642, ISSN: 1944-0049, DOI: 10.1080/19440049.2014.976279
- GIRIDHARAN GOKULRANGAN ET AL: "DNA Aptamer-Based Bioanalysis of IgE by Fluorescence Anisotropy", ANALYTICAL CHEMISTRY, vol. 77, no. 7, 1 April 2005 (2005-04-01), pages 1963-1970, XP055262658, US ISSN: 0003-2700, DOI: 10.1021/ac0483926
- WENDY A LEA ET AL: "Fluorescence polarization assays in small molecule screening", EXPERT OPINION ON DRUG DISCOVERY, vol. 6, no. 1, 1 January 2011 (2011-01-01) , pages 17-32, XP055208631, ISSN: 1746-0441, DOI: 10.1517/17460441.2011.537322
- STIJLEMANS B ET AL: "Efficient targeting of conserved cryptic epitopes of infectious agents by single domain antibodies. African trypanosomes as paradigm", JOURNAL OF BIOLOGICAL CHEMISTRY, AMERICAN SOCIETY FOR BIOCHEMISTRY AND MOLECULAR BIOLOGY, US, vol. 279, no. 2, 3 October 2003 (2003-10-03), pages 1256-1261, XP002367544, ISSN: 0021-9258, DOI: 10.1074/JBC.M307341200
- M. Rajabi Bazl ET AL: "Production of Chimeric Recombinant Single Domain Antibody-Green Fluorescent Fusion Protein in Chinese Hamster Ovary Cells", Hybridoma, vol. 26, no. 1, 1 February 2007 (2007-02-01), pages 1-9, XP55575718, US ISSN: 1554-0014, DOI: 10.1089/hyb.2006.037

## Description

### Introduction

Immunofluorescence is a technique used for light microscopy with a fluorescence microscope and is used primarily on microbiological samples. This technique uses the specificity of antibodies to their antigen to target fluorescent dyes to specific biomolecule targets within a cell, and therefore allows visualisation of the distribution of the target molecule through the sample. Immunofluorescence is a widely used example of immunostaining and is a specific example of immunohistochemistry that makes use of fluorophores to visualise the location of the antibodies.

Fluorescent polarisation immunoassays are described in Nielsen et al. (Methods 22, 71-76, 2000) and can be employed for the rapid and accurate detection of antibody or antigen. The assay is based on the principle that small molecules rotate faster than larger molecules in solution, and that the rotation rate can be determined by fluorescent polarisation. When an antibody in a sample is incubated with a specific antigen for the antibody that is labelled with a fluorescent probe, the presence of antibody-antigen complex in the sample can be detected by means of fluorescence polarisation. A problem with this technique is that for detection of a given antibody, a specific antigen for that antibody is required which makes the assay expensive for many companies. A further problem with this technique is that is not suitable for quantitative detection of antibody in a sample.

Min Chen et al. (Food additives & Contaminants: part A, vol. 31(12), pp. 1959-1967 (2014) describes a method kit and conjugate for determining the abundance of an antibiotic in milk by reacting the milk with a bi-specific single-chain antibody and FITC-labelled target molecules in a reaction vessel and correlating the competitive reaction with the quantity of the target molecule. Giridhara Gokulrangan et al. (Analytical Chemistry, vol. 77(1), pp. 17-32 (2011) describes a method of determining the abundance of a target antibody by fluorescence polarisation wherein the target antibody binding probe is an aptamer linked to a fluorophore.

It is an object of the invention to overcome at least one of the above-referenced problems.

### Brief Description of the Invention

The invention is based on the finding that the presence or abundance of a target molecule in a sample can be determined using fluorescence polarisation and a tracer comprising a fluorescent dye conjugated to a single domain antibody, where the small size of the single domain antibody allows for highly sensitive quantification of the target molecule in a sample using a fluorescent polarisation format.

In a first aspect, the invention provides a method for determining the presence or abundance of a target molecule in a liquid sample comprising the steps of:
- incubating in a reaction chamber the liquid sample with a target molecule-binding probe comprising a single domain antibody conjugated to a fluorescent probe to provide a reaction mixture, wherein the single chain antibody is capable of binding selectively to the target molecule;
- assaying the reaction mixture in the reaction chamber for fluorescence polarisation to detect a change in polarisation between excitation and emission light; and
- correlating the change in polarisation with the presence or abundance of target molecule in the sample.

In one embodiment, the target molecule is or comprises a polyamino acid such as a protein, polypeptide or peptide. In one embodiment, the target protein is a target antibody. In one embodiment, the single chain antibody is capable of binding selectively to a constant region of the target antibody. In one embodiment, the single chain antibody is capable of binding selectively to a variable region of the target antibody. In one embodiment, the single chain antibody is capable of binding selectively to a paratope of the target antibody. In one embodiment, the single chain antibody is capable of binding selectively to a specific isotype of antibody (*i.e.* capable of binding to all IgE antibodies in a sample). In one embodiment, the target antibody is an IgE antibody. In one embodiment, the target antibody is an IgG antibody. In one embodiment, the target antibody is an IgM antibody. In one embodiment, the target antibody is an IgA antibody. In one embodiment, the target antibody is an IgA antibody. In one embodiment, the target antibody is an IgY antibody. In one embodiment, the target antibody is an IgW antibody. In one embodiment, the target molecule is an antigen. In one embodiment, the antigen is a microbial-specific antigen. In one embodiment, the antigen is a disease-specific antigen. In one embodiment, the antigen is a cell-specific antigen. In one embodiment, the antigen is a phenotype-specific antigen.

In one embodiment, the target molecule is or comprises a target oligosaccharide.

In one embodiment, the target molecule is or comprises a target nucleic acid.

In one embodiment, the target molecule is expressed by a cell. In one embodiment, the reaction mixture comprises a cell culture wherein the cell culture expresses the target molecule. In one embodiment, the cell culture comprises monoclonal antibody producing cells and in which the monoclonal antibody is the target molecule. In one embodiment, the cell is a eukaryotic or prokaryotic producer cell.

Preferably, the reaction chamber is a well of a multiwall plate.

Preferably, the fluorescent probe is a long-life fluorescent probe.

In one embodiment, the method is a rapid method for determining the abundance of a target molecule in a liquid sample. In one embodiment, the method is a high-throughput method for determining the abundance of a target molecule in a liquid sample.

In one embodiment, the plurality of cell culture samples comprises a panel of clonal producer cells. Thus, the invention also relates to a rapid, high-throughput, method of determining antibody titre in a panel of clonal producer cells in which the antibody is the target molecule.

Typically, method employs a fluorescence polarisation analyser capable of performing a fluorescence polarisation assay on a plurality of wells of the microtitre plate simultaneously.

The invention also provides a kit typically suitable for performing the method of the invention and comprising (a) a target molecule-binding probe comprising a single domain antibody conjugated to a fluorescent probe and (b) a fluorescence polarisation analyser. In one embodiment, the probe comprises a fluorescent probe having a fluorescence lifetime of at least 3ns. In one embodiment, the fluorescence polarisation analyser is configured to detect changes in fluorescent polarisation in a plurality of wells of a microtiter plate simultaneously.

### Brief Description of the Figures

The invention will be more clearly understood from the following description of an embodiment thereof, given by way of example only, with reference to the accompanying drawings, in which:-
**Figure 1** is a graph showing detection of nanobody binding of IgE, where 0.005 mg/L of labeled nanobody in PBS/BSA solution was added to human IgE. Polarisation signals for each concentration were measured.
**Figure 2****:** is a graph showing lack of detection of nanobody binding of IgG, where 0.005 mg/L of labeled nanobody in PBS/BSA solution was added to human IgG. Polarisation signals for each concentration were measured.

### Detailed Description of the Invention

"Target molecule-binding probe" means a conjugate of single domain antibody and a fluorescent probe in which in the conjugated state the single domain antibody is capable of binding selectively to the target molecule and in which the fluorescent probe is capable of re-emitting light upon excitation. Conjugation can be achieved by covalent bonding using, for example, a stable linker which may be cleavable or non-cleavable. Specific methods of coupling antibodies, including single domain antibodies and single chain antibodies, to a separate entity will be well known to those skilled in the art and are described in, for example:
Chakravarty et al., Theranostics 2014; 4(4): 386-398;
ADC Review / Journal of Antibody-drug Conjugates: Stable Linker (technologies), May 23, 2013;
Methods Mol Biol. 2013; 1045:1-27. doi: 10.1007/978-1-62703-541-5_1;
"Cell killing by antibody-drug conjugates". Cancer letters 255(2): 232-40.doi:10.1016/j.canlet.2007.04.010. PMID 17553616;
"New method of peptide cleavage based on Edman degradation". Molecular diversity 17(3): 605-11. doi:10.1007/s11030-013-9453-y. PMC 3713267. PMID 23690169;
"Synthesis of site-specific antibody-drug conjugates using unnatural amino acids". Proceedings of the National Academy of Sciences 109(40): 16101-6.doi:10.1073/pnas.1211023109. PMC 3479532.PMID 22988081;
"Current methods for the synthesis of homogenous antibody-drug conjugates" Biotechnology Advances (33) Issue 6, Part 1 (see especially Table 1);
Badescu et al., Bioconj. Chem. 25 (2014), 1124-1136;
Dennler et al. Bioconj. Chem. 25 (2014), 569-578;
Drake et al. Bioconj. Chem. 25 (2014), 1131-1341;
Hofer et al. Biochemistry, 48 (2009), 12047-12057;
Senter et al. Nat. Biotechnol. 30 (2012) 631-637;
Shumacher et al. Org. Biomol. Chem. 12 (2104), 7261-7269;
Strop et al. Chem. Biol. 20 (2013), 161-167;
Zhou et al. MAbs 6 (2014), 1190-1200; and
Zimmerman *et al.* 25 (2014), 351-361.

Site-specific conjugates of single domain antibodies and fluorescent dyes may be obtained commercially, for example from the University of Utrecht Nanobody Facility (http://www.uu.nl/en/research/cell-biology/facilities/utrecht-nanobody-facility-unf).

In one embodiment, the single domain antibody is engineered to comprise an attachment moiety for attachment of a fluorescent probe to the single domain antibody. In one embodiment, the single domain antibody is genetically engineered to comprise the attachment moiety. The synthesis of antibodies engineered to comprise attachment moieties for, inter alia, dyes is described in International Patent Application No: PCT/NL97/00653.

In this specification, the term "single domain antibody" or "sdAb" or "nanobody" is an antibody fragment consisting of a single monomeric variable antibody domain that is capable of binding selectively to a specific antigen, for example a specific protein, a specific IgG molecule, or to IgE molecules (Harmsen et al., Appl. Micrbiol. Biotechnol. 77 (1) 13-22). They typically have a molecular weight of less than 18kDa. In one embodiment, the single domain antibody has a molecular weight of about 10-15 kDa. In one embodiment, the single domain antibody has a molecular weight of about 12-15 kDa. They are generally obtained from heavy chain antibodies, or from conventional antibodies. Obtaining single domain antibodies from heavy chain antibodies involves immunization of certain animals with a desired antigen, isolation of the mRNA coding for the heavy chain antibody, and generating a gene library of single domain antibodies by means of reverse transcription and PCR (Ghahroudi et al., FEBS Letters, 414 (3) 521-526). In certain cases, the initial immunization step is not required, resulting in the production of a naive gene library of single domain antibodies (Saerens et al, Current Opinion in Pharmacology, 8 (5) 600-608). Single chain antibodies can be obtained from conventional antibodies, such as human or murine IgG antibodies, in a process involving the generation of a gene library from an immunized or naive donor (Holt et al, Trends in Biotechnology 21 (11) 484-490, and Borrebaeck et al, Nature Biotechnology 20 (12)). In one embodiment, the conventional antibody is a human antibody. In one embodiment, the conventional is a humanised antibody. Single chain antibodies may be obtained from commercial sources, for example from Creative Biolabs (www.creative-biolabs.com) and Precision Antibody (www.precisionantibody.com). In one embodiment, the single domain antibody is capable of binding selectively to a target antibody. In one embodiment, the single domain antibody is capable of binding selectively to a specific antibody isotype (for example binding to all IgE antibodies in a sample). In one embodiment, the single domain antibody is capable of binding selectively to a specific antibody (for example, by binding specifically to the paratope of the antibody). In one embodiment, the single domain antibody is specific to an expression product of a biopharmaceutical producer cell. Examples of such expression products include monoclonal antibodies, fusion proteins. Examples of biopharmaceutical producer cells include chinese hamster ovary (CHO) cells and baby hamster kidney (BHK) cells). In one embodiment, the single chain antibody is capable of binding selectively to a constant region of the target antibody. In one embodiment, the single chain antibody is capable of binding selectively to a variable region of the target antibody (for example, a hypervariable region). In one embodiment, the single chain antibody is capable of binding selectively to a paratope of the target antibody. In one embodiment, the single chain antibody is capable of binding selectively to a specific isotype of antibody (*i.e.* capable of binding to, for example, all IgE antibodies in a sample). In one embodiment, the target antibody is an IgE antibody. In one embodiment, the target antibody is an IgG antibody. In one embodiment, the target antibody is an IgM antibody. In one embodiment, the target antibody is an IgD antibody. In one embodiment, the target antibody is an IgA antibody. In one embodiment, the target antibody is an IgY antibody. In one embodiment, the target antibody is an IgW antibody.

In this specification, the term "antibody" and "target antibody" should be understood to mean an immunoglobulin, for example an IgG, IgE, IgA, IgD, IgM, IgY or IgW immunoglobulin in a monoclonal or polyclonal form, or a fragment thereof, in a humanised or non-humanised.

In this specification, the term "assaying the sample in the reaction chamber for fluorescence polarisation" should be understood to mean exciting the sample with (vertical or horizontal) plane polarised light at a wavelength corresponding to an excitation wavelength of the fluorescent probe, and detecting light intensity emitted by the fluorescent probe at an appropriate emission wavelength both in a vertical and horizontal plane. The degree to which the emission intensity moves from the excitation plane (*i.e.* vertical) to an opposite plane (*i.e.* horizontal) - *i.e.* the change in polarisation between excitation and emission light - is a function of the degree of rotation of the fluorescent probe. When the target molecule-binding probe is bound to target molecule, the complex will rotate slower than the target molecule-binding probe, resulting in an increase in polarisation of emitted light which can be quantified.

The term "correlating the change in polarisation with the abundance of the target molecule" should be understood to mean the abundance of the target molecule in the sample based on the change in polarisation of light emitted by the fluorescent probe. Methods for calculating the target molecule abundance include inferring the abundance from a standard curve of known concentrations of target molecule or by calculating dissociation constants and necessary parameters, and inferring concentration from first principles.

In this specification, the term "rapid" should be understood to mean that the method can be carried out in 12 hours or less, preferably less than 10, 8, 6, 4, 2 or 1 hours. In this specification, the term "high-throughput" should be understood to mean a method in which a large number of samples, for example at least 20, 50, 90, 120, i.e. 20-500, can be assayed simultaneously.

The term "antibody titer" as used herein refers to the amount of antobody, generally a recombinant antibody, and ideally a recombinant monoclonal antibody, present in the sample at a given time point. Typically, the sample is a cell culture sample. Preferably, the sample is supernatant derived from a cell culture sample. The titer may be quantified in absolute or relative terms. Generally, titre is referred to as weight of product per volume of culture - grams per litre (g/L) is a common metric.

The term "sample" as used herein should be understood to mean a liquid sample, for example a cell culture sample or supernatant derived from a cell culture sample. Preferably, the cells are producer cells (*i.e.* cells genetically modified to produce a recombinant protein), for example prokaryotic producer cells (i.e. E. Coli) or eukaryotic producer cells (i.e. CHO cells). Examples of both prokaryothic and eukaryotic producer cells will be known to a person skilled in the art.

In this specification, the term "fluorescent probe" or "fluorescent dye" or "fluorophore" should be understood to mean a fluorescent chemical that can absorb light of a specific wavelength and emit light of a different, typically longer, wavelength. Examples of fluorescent probes will be known to those skilled in the art, and include fluorescent proteins such as GFP, YFP and RFP, and non-protein organic fluorophores including tetrapyrrole derivatives, pyrene derivatives, xanthene derivatives, and cyanine derivatives. Specific examples of fluorescent probes are provided below. In this specification, the term "long life fluorescent probe" should be understood to mean a fluorescent probe having a fluoresence lifetime of at least 4, 5, 10, 12, 14, 16, 18 or 20 nanoseconds. Preferably, the fluorescent probe has a fluoresence lifetime of 4-100 ns, 4-50 ns, 4-40 ns, 4-30 ns or 4-25 ns, typically 10-100 ns, 10-50 ns, 10-40 ns, 10-30 ns or 5-25 ns, and ideally 15-100 ns, 15-50 ns, 15-40 ns, 15-30 ns or 15-25 ns. Examples of fluorescent probes are described at www.Fluorophores.tugraz.at/substance and a specific sample is provided in the Table below, including long-life fluorophores.

| Fluorophore | Lifetime [ns] | Solvent | Excitation max [nm] | Emission max [nm] |
|---|---|---|---|---|
| **5-Hydroxytryptamine** | | | 370-415 | 520-540 |
| **ATTO 565** | 3.4 | Water | 561 | 585 |
| **ATTO 655** | 3.6 | Water | 655 | 690 |
| **Acridine Orange** | 2 | PB pH 7.8 | 500 | 530 |
| **Acridine Yellow** | | | 470 | 550 |
| **Alexa Fluor 488** | 4.1 | PB pH 7.4 | 494 | 519 |
| **Alexa Fluor 532** | | | 530 | 555 |
| **Alexa Fluor 546** | 4 | PB pH 7.4 | 554 | 570 |
| **Alexa Fluor 633** | 3.2 | Water | 621 | 639 |
| **Alexa Fluor 647** | 1 | Water | 651 | 672 |
| **Alexa Fluor 680** | 1.2 | PB pH 7.5 | 682 | 707 |
| **BODIPY 500/510** | | | 508 | 515 |
| **BODIPY 530/550** | | | 534 | 554 |
| **BODIPY FL** | 5.7 | Methanol | 502 | 510 |
| **BODIPY TR-X** | 5.4 | Methanol | 588 | 616 |
| **Cascade Blue** | | | 375 | 410 |
| **Coumarin 6** | 2.5 | Ethanol | 460 | 505 |
| **CY2** | | | 489 | 506 |
| **CY3B** | 2.8 | PBS | 558 | 572 |
| **CY3** | 0.3 | PBS | 548 | 562 |
| **CY3.5** | 0.5 | PBS | 581 | 596 |
| **CY5** | 1 | PBS | 646 | 664 |
| **CY5.5** | 1 | PBS | 675 | 694 |
| **Dansyl** | 10 | | 340 | 520 |
| **DAPI** | 0.16 | TRIS/EDTA | 341 | 496 |
| **DAPI + ssDNA** | 1.88 | TRIS/EDTA | 358 | 456 |
| **DAPI + dsDNA** | 2.2 | TRIS/EDTA | 356 | 455 |
| **DPH** | | | 354 | 430 |
| **Erythrosin** | | | 529 | 554 |
| **Ethidium Bromide - no DNA** | 1.6 | TRIS/EDTA | 510 | 595 |
| **Ethidium Bromide + ssDNA** | 25.1 | TRIS/EDTA | 520 | 610 |
| **Ethidium Bromide + dsDNA** | 28.3 | TRIS/EDTA | 520 | 608 |
| **FITC** | 4.1 | PB pH 7.8 | 494 | 518 |
| **Fluorescein** | 4 | PB pH 7.5 | 495 | 517 |
| **FURA-2** | | | 340-380 | 500-530 |
| **GFP** | 3.2 | Buffer pH 8 | 498 | 516 |
| **Hoechst 33258 - no DNA** | 0.2 | TRIS/EDTA | 337 | 508 |
| **Hoechst 33258 + ssDNA** | 1.22 | TRIS/EDTA | 349 | 466 |
| **Hoechst 33258 + dsDNA** | 1.94 | TRIS/EDTA | 349 | 458 |
| **Hoechst 33342 - no DNA** | 0.35 | TRIS/EDTA | 336 | 471 |
| **Hoechst 33342 + ssDNA** | 1.05 | TRIS/EDTA | 350 | 436 |
| **Hoechst 33342 + dsDNA** | 2.21 | TRIS/EDTA | 350 | 456 |
| **HPTS** | 5.4 | PB pH 7.8 | 454 | 511 |
| **Indocyanine Green** | 0.52 | Water | 780 | 820 |
| **Laurdan** | | | 364 | 497 |
| **Lucifer Yellow** | 5.7 | Water | 428 | 535 |
| **Nile Red** | | | 485 | 525 |
| **Oregon Green 488** | 4.1 | Buffer pH 9 | 493 | 520 |
| **Oregon Green 500** | 2.18 | Buffer pH 2 | 503 | 522 |
| **Oregon Green 514** | | | 511 | 530 |
| **Prodan** | 1.41 | Water | 361 | 498 |
| **Pyrene** | > 100 | Water | 341 | 376 |
| **Rhodamine 101** | 4.32 | Water | 496 | 520 |
| **Rhodamine 110** | 4 | Water | 505 | 534 |
| **Rhodamine 123** | | | 505 | 534 |
| **Rhodamine 6G** | 4.08 | Water | 525 | 555 |
| **Rhodamine B** | 1.68 | Water | 562 | 583 |
| **Ru(bpy)₃[PF₆]₂** | 600 | Water | 455 | 605 |
| **Ru(bpy)₂(dcpby)[PF₆]₂** | 375 | Buffer pH 7 | 458 | 650 |
| **SeTau-380-NHS** | 32.5 | Water | 270 | 480 |
| **SeTau-404-NHS** | 9.3 | Water | 402 | 515 |
| **SeTau-405-NHS** | 9.3 | Water | 405 | 518 |
| | | | 340 | |
| **SeTau-425-NHS** | 26.2 | Water | 425 | 545 |
| **SITS** | | | 336 | 438 |
| **SNARF** | | | 480 | 600-650 |
| **Stilbene SITS, SITA** | | | 365 | 460 |
| **Texas Red** | 4.2 | Water | 589 | 615 |
| **TOTO-1** | 2.2 | Water | 514 | 533 |
| **YOYO-1 no DNA** | 2.1 | TRIS/EDTA | 457 | 549 |
| **YOYO-1 + ssDNA** | 1.67 | TRIS/EDTA | 490 | 510 |
| **YOYO-1 + dsDNA** | 2.3 | TRIS/EDTA | 490 | 507 |
| **YOYO-3** | | | 612 | 631 |

The term "producer cell" refers to a cell that is employed to generate a specific desired protein. Generally, the cell is genetically modifed to include one or multiple copies of a transgene encoding the desired protein which is generally under the control of a specific promotor. Thus, the specific protein is usually a recombinant protein. Producer cells are well known in the art, and include for example Chinese hamster ovary (CHO) cells or baby hamster kidney (BHK) cells. Ideally, the producer cell is a CHO cell. Typically, the producer cell is a monoclonal antibody producer cell.

In this specification, the term "microtiter plate" refers to a plate having a multiplicity of wells, for example at least 10, 15, 20, 30, 40, 50, 60, 70, 80, 90 or 100 wells. In one embodiment, the plate may be a solid, non-flexible plate and alternatively may be provided as a roll of flexible material.

### Experimental

### Labelling of Nanobody

An anti-human IgE nanobody was purchased from Creative Biolabs, US. This was N terminal labeled in PBS using FITC (Sigma, UK) at a molar ratio of 8:1 dye to protein using a 1 hour incubation time at room temperature. This was purified using a Sephedex desalting column (GE, UK) and concentrated using a 3KDa cutoff spin column (Amicon, UK).

An anti-human IgG nanobody was purchased from Creative Biolabs, US. This was N terminal labeled in PBS using FITC (Sigma, UK) at a molar ratio of 8:1 dye to protein using a 1 hour incubation time at room temperature. This was purified using a Sephedex desalting column (GE, UK) and concentrated using a 3KDa cutoff spin column (Amicon, UK).

An anti-human IgD nanobody was purchased from Creative Biolabs, US. This was N terminal labeled in PBS using FITC (Sigma, UK) at a molar ratio of 8:1 dye to protein using a 1 hour incubation time at room temperature. This was purified using a Sephedex desalting column (GE, UK) and concentrated using a 3KDa cutoff spin column (Amicon, UK).

An anti-human IgA nanobody was purchased from Creative Biolabs, US. This was N terminal labeled in PBS using FITC (Sigma, UK) at a molar ratio of 8:1 dye to protein using a 1 hour incubation time at room temperature. This was purified using a Sephedex desalting column (GE, UK) and concentrated using a 3KDa cutoff spin column (Amicon, UK).

An anti-human IgM nanobody was purchased from Creative Biolabs, US. This was N terminal labeled in PBS using FITC (Sigma, UK) at a molar ratio of 8:1 dye to protein using a 1 hour incubation time at room temperature. This was purified using a Sephedex desalting column (GE, UK) and concentrated using a 3KDa cutoff spin column (Amicon, UK).

An anti-human IgY nanobody was purchased from Creative Biolabs, US. This was N terminal labeled in PBS using FITC (Sigma, UK) at a molar ratio of 8:1 dye to protein using a 1 hour incubation time at room temperature. This was purified using a Sephedex desalting column (GE, UK) and concentrated using a 3KDa cutoff spin column (Amicon, UK).

An anti-human IgW nanobody was purchased from Creative Biolabs, US. This was N terminal labeled in PBS using FITC (Sigma, UK) at a molar ratio of 8:1 dye to protein using a 1 hour incubation time at room temperature. This was purified using a Sephedex desalting column (GE, UK) and concentrated using a 3KDa cutoff spin column (Amicon, UK).

### Demonstration of fluorescence polarization to quantify IgE in solution.

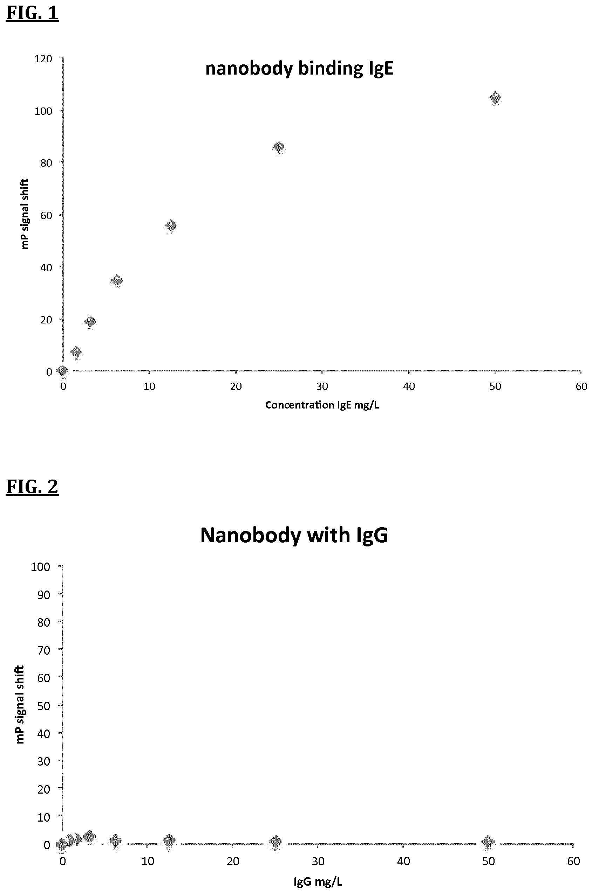

For proof of concept, the labeled nanobody was diluted to a concentration of 0.005 mg/ml in PBS with 1mg/ml BSA to prevent non-specific binding. A standard curve of human IgE was set up in PBS with the following concentrations in mg/L 50,25,12.5,6.25, 3.125,0. For the assay, 60ul of FITC nanobody was added to 60ul of diluted IgE and this was incubated in the dark for 30 minutes prior to reading the fluorescence polarization. From Figure 1, it is evident that this approach can be used to quantify IgE in solution from the shift in polarization signal.

### Demonstration of nanobody Isotype specificity

For proof of specificity, the above was repeated using human IgG. Anti-IgE nanobody-FITC was diluted to a concentration of 0.005 mg/ml in PBS with 1mg/ml BSA to prevent non-specific binding. A standard curve of human IgG was set up in PBS with the following concentrations in mg/L 50,25,12.5,6.25, 3.125,0. 60ul of FITC nanobody was added to 60ul of diluted IgG and this was incubated in the dark for 30 minutes prior to reading the fluorescence polarization. From Figure 2, it is evident that there is no cross-reactivity with another isotype of immunoglobulin, thus demonstrating the specificity for IgE.

### Demonstration of fluorescence polarization to quantify IgA, IgD, IgG, IgM, IgY and IgW in solution.

For proof of concept, the labelled anti-A, D, G, M, Y and W nanobody (respectively) was diluted to a concentration of 0.005 mg/ml in PBS with 1mg/ml BSA to prevent non-specific binding. A standard curve for each of human IgA, IgD, IgG, IgM, IgY and IgW was set up in PBS with the following concentrations in mg/L 50, 25, 12.5, 6.25, 3.125, 0. For the assay, 60ul of FITC anti-A, D, G, M, Y and W nanobody (repectively) was added to each of 60ul of diluted IgA, IgD, IgG, IgM, IgY and IgW, and these was incubated in the dark for 30 minutes prior to reading the fluorescence polarization. This approach can be used to quantify IgA, IgD, IgG, IgM, IgY and IgW in solution from the shift in polarization signal.

### Demonstration of nanobody Isotype specificity

For proof of specificity, the above was repeated using human IgE. Anti-IgA, Anti-IgD, Anti-IgG, Anti-IgM, Anti-IgY and Anti-IgW nanobody-FITC were diluted, separately, to a concentration of 0.005 mg/ml in PBS with 1mg/ml BSA to prevent non-specific binding. A standard curve of human IgE was set up in PBS with the following concentrations in mg/L 50, 25, 12.5, 6.25, 3.125, 0. 60ul of each FITC nanobody was added separately to 60ul of diluted IgE and these were incubated in the dark for 30 minutes prior to reading the fluorescence polarization. This will show that there is no cross-reactivity with another isotype of immunoglobulin, thus demonstrating the specificity for each of IgA, IgD, IgG, IgM, IgY and IgW.

## Claims

1. A method for determining the abundance of a target molecule in a liquid sample comprising the steps of:
- incubating in a reaction chamber the liquid sample with a target molecule-binding probe comprising a single domain antibody conjugated to a fluorescent probe to provide a reaction mixture, wherein the single chain antibody is capable of binding selectively to the target molecule;
- assaying the reaction mixture in the reaction chamber for fluorescence polarisation to detect a change in polarisation between excitation and emission light; and
- correlating the change in polarisation with the abundance of target molecule in the sample.

2. A method as claimed in Claim 1 in which the fluorescent probe has a lifetime of at least 4ns.

3. A method as claimed in any preceding Claim in which the single domain antibody is capable of specifically binding to a hypervariable region of a target antibody.

4. A method as claimed in any preceding Claim in which the single domain antibody is capable of specifically binding to a constant region of a target antibody.

5. A method as claimed in any preceding Claim which is a rapid, high-throughput, method of simultaneously determining the abundance of at least one target molecule in a plurality of liquid samples, in which each liquid sample is individually incubated in a well of a microtiter plate.

6. A method as claimed in Claim 5 in which the method employs a fluorescence polarisation analyser capable of assaying a plurality of wells of the microtitre plate for fluorescent polarisation simultaneously.

7. A method as claimed in any preceding Claim in which the or each liquid sample comprises a cell culture.

8. A method according to Claim 7 in which the target molecule is a recombinant protein and in which the cell culture comprises producer cell engineered to overexpress the recombinant protein.

9. A method according to Claim 7 in which the recombinant protein is a monoclonal antibody.

10. A kit typically suitable for performing the method of any of Claims 1 to 9 and comprising (a) a target molecule-binding probe comprising a single domain antibody conjugated to a fluorescent probe and (b) a fluorescence polarisation analyser.

11. A kit according to Claim 10 in which the fluorescent probe is a long-lifetime fluorescent probe.

12. A kit according to Claim 10 or 11 in which the fluorescence polarisation analyser is configured to detect changes in fluorescent polarisation in a plurality of wells of a microtiter plate simultaneously.

## Patentansprüche

1. Verfahren für die Bestimmung der Häufigkeit eines Zielmoleküls in einer flüssigen Probe, das die folgenden Schritte umfasst:
- Inkubieren in einer Reaktionskammer der flüssigen Probe mit einer das Zielmolekül bindenden Sonde, die einen Einzeldomänenantikörper, der zu einer fluoreszierenden Sonde konjugiert ist, umfasst, um eine Reaktionsmischung bereitzustellen, wobei der Einzelkettenantikörper selektiv zu dem Zielmolekül binden kann;
- Untersuchen der Reaktionsmischung in der Reaktionskammer auf Fluoreszenzpolarisation, um eine Änderung der Polarisation zwischen Anregungs- und Emissionslicht zu detektieren; und
- Korrelieren der Änderung der Polarisation mit der Häufigkeit des Zielmoleküls in der Probe.

2. Verfahren nach Anspruch 1, in dem die fluoreszierende Sonde eine Lebensdauer von mindestens 4 ns hat.

3. Verfahren nach einem vorstehenden Anspruch, in dem der Einzeldomänenantikörper spezifisch zu einer hypervariablen Region eines Zielantikörpers binden kann.

4. Verfahren nach einem vorstehenden Anspruch, in dem der Einzeldomänenantikörper spezifisch zu einer konstanten Region eines Zielantikörpers binden kann.

5. Verfahren nach einem vorstehenden Anspruch, das ein schnelles Hochdurchsatz-Verfahren zur gleichzeitigen Bestimmung der Häufigkeit von mindestens einem Zielmolekül in einer Vielzahl von flüssigen Proben ist, in dem jede flüssige Probe einzeln in einem Well einer Mikrotiterplatte inkubiert wird.

6. Verfahren nach Anspruch 5, in dem das Verfahren einen Fluoreszenzpolarisationsanalysator einsetzt, der gleichzeitig eine Vielzahl von Wells der Mikrotiterplatte auf Fluoreszenzpolarisation untersuchen kann.

7. Verfahren nach einem vorstehenden Anspruch, in dem die oder jede flüssige Probe eine Zellkultur umfasst.

8. Verfahren nach Anspruch 7, in dem das Zielmolekül ein rekombinantes Protein ist und in dem die Zellkultur eine Erzeugerzelle umfasst, die zur Überexpression des rekombinanten Proteins konstruiert ist.

9. Verfahren nach Anspruch 7, in dem das rekombinante Protein ein monoklonaler Antikörper ist.

10. Kit, der typischerweise für die Durchführung des Verfahrens nach einem der Ansprüche 1 bis 9 geeignet ist und Folgendes umfasst:
(a) eine das Zielmolekül bindende Sonde, die einen Einzeldomänenantikörper, der zu einer fluoreszierenden Sonde konjugiert ist, umfasst und (b) einen Fluoreszenzpolarisationsanalysator.

11. Kit nach Anspruch 10, in dem die fluoreszierende Sonde eine fluoreszierende Sonde mit langer Lebensdauer ist.

12. Kit nach Anspruch 10 oder 11, in dem der Fluoreszenzpolarisationsanalysator zur gleichzeitigen Detektion von Änderungen der Fluoreszenzpolarisation in einer Vielzahl von Wells einer Mikrotiterplatte konfiguriert ist.

## Revendications

1. Méthode de détermination de l'abondance d'une molécule cible dans un échantillon liquide, comprenant les étapes consistant à :
- incuber, dans une chambre de réaction, l'échantillon liquide avec une sonde de fixation de molécule cible, comprenant un anticorps à domaine unique conjugué à une sonde fluorescente afin de fournir un mélange réactionnel, où l'anticorps à chaîne unique est capable de se fixer sélectivement à la molécule cible ;
- le dosage du mélange réactionnel dans la chambre de réaction de polarisation de fluorescence afin de détecter un changement de polarisation entre la lumière d'excitation et d'émission ; et
- la corrélation du changement de polarisation avec l'abondance de la molécule cible dans l'échantillon.

2. Méthode selon la revendication 1, dans laquelle la sonde fluorescente possède une durée de vie d'au moins 4 ns.

3. Méthode selon l'une quelconque des revendications précédentes, dans laquelle l'anticorps à domaine unique est capable de se fixer spécifiquement à une région hyper variable d'un anticorps cible.

4. Méthode selon l'une quelconque des revendications précédentes, dans laquelle l'anticorps à domaine unique est capable de se fixer spécifiquement à une région constante d'un anticorps cible.

5. Méthode selon l'une quelconque des revendications précédentes, qui est une méthode rapide, à haut débit, de détermination simultanée de l'abondance d'au moins une molécule cible dans une pluralité d'échantillons liquides, où chaque échantillon liquide est incubé individuellement dans un puits d'une plaque de microtitration.

6. Méthode selon la revendication 5, où la méthode emploie un analyseur par polarisation de fluorescence capable de doser de manière simultanée une pluralité de puits de la plaque de microtitration de polarisation de fluorescence.

7. Méthode selon l'une quelconque des revendications précédentes, dans laquelle le ou chaque échantillon liquide comprend une culture cellulaire.

8. Méthode selon la revendication 7, dans laquelle la molécule cible est une protéine recombinée et où la culture cellulaire comprend des cellules productrices modifiées afin de surexprimer la protéine recombinée.

9. Méthode selon la revendication 7, dans laquelle la protéine recombinée est un anticorps monoclonal.

10. Kit typiquement convenable pour la mise en œuvre de la méthode selon l'une quelconque des revendications 1 à 9, et comprenant (a) une sonde de fixation de molécule cible, comprenant un anticorps à domaine unique conjugué à une sonde fluorescente et (b) un analyseur par polarisation de fluorescence.

11. Kit selon la revendication 10, dans lequel la sonde fluorescente est une sonde fluorescente à longue durée de vie.

12. Kit selon la revendication 10 ou 11, dans lequel l'analyseur par polarisation de fluorescence est configuré afin de détecter de manière simultanée des changements de polarisation de fluorescence dans une pluralité de puits d'une plaque de microtitration.
